# EUROPEAN PATENT APPLICATION

(11) **EP 3 603 692 A1**
(43) Date of publication of application: **05.02.2020**
(21) Application number: 18774235.8
(22) Date of filing: 12.01.2018
(51) Int. Cl.: A61M 1/28, A61L 2/10, A61M 25/00

(54) **LIGHT INTRODUCTION DEVICE AND STERILIZATION SYSTEM**

(30) Priority: 31.03.2017 JP 2017070434
(71) Applicant: Nikkiso Co., Ltd., Shibuya-ku, Tokyo 150-6022 (JP)
(72) Inventor: SUGIMOTO Koichi, Tokyo 150-6022 (JP); NAKAMA Takahiro, Tokyo 150-6022 (JP); ASANO Hideki, Tokyo 150-6022 (JP)
(74) Representative: Algemeen Octrooi- en Merkenbureau B.V.
(86) International application number: PCT/JP2018/000702
(87) International publication number: WO 2018/179676

(57) **Abstract**

A light introduction device (3) has an inner cavity allowing passage of at least one of a substance introduced into a human body or a substance discharged from the human body. Light is introduced from part of an outer surface of a section, which is arranged outside the human body, of a catheter (2) as a medical conduit having a light-permeable thick portion.

## Description

### Technical Field

The present invention relates to a light introduction device and a sterilization system.

### Background Art

An instantaneous sterilization/disinfection unit configured to sterilize, using light, a catheter inserted into a patient and/or a skin therearound is disclosed (see Patent Literature 1 below). In the instantaneous sterilization/disinfection unit, a light source configured to emit ultraviolet light (UV) is arranged above an insertion section of the catheter and/or the catheter around the insertion section, and the light source irradiates the insertion section of the catheter and/or the periphery of the insertion section with the UV.

### Citation List

### Patent Literature

[Patent Literature 1] JP2009-261957 A

### Summary of Invention

However, in the instantaneous sterilization/disinfection unit of Patent Literature 1 above, tendency shows that a catheter portion surrounded by a human tissue is less irradiated with the UV emitted from the outside of the patient. Specifically, tendency shows that a back side of the catheter portion surrounded by the human tissue on the opposite side of the side provided with the light source is less irradiated with the ultraviolet light. For this reason, there are concerns that deactivation of bacteria present between the catheter and the human tissue is insufficient, and a demand for deactivation has been increased. Moreover, a demand for deactivation of bacteria present in liquid introduced into the catheter has been also increased. However, in the instantaneous sterilization/disinfection unit of Patent Literature 1 above, the patient is merely directly irradiated with light, and the bacteria present between the catheter and the human tissue and the bacteria present in the liquid in the catheter cannot be deactivated. Thus, a device configured to irradiate bacteria with light through a medical conduit to deactivate the bacteria has been demanded.

Thus, the present invention provides a light introduction device and a sterilization system configured so that bacteria can be irradiated with light through a medical conduit to enhance a sterilization effect against a substance contacting the medical conduit.

The light introduction device of the present invention is characterized by including an inner cavity allowing passage of at least one of a substance introduced into a human body or a substance discharged from the human body. Light is introduced from part of an outer surface of a section, which is arranged outside the human body, of a medical conduit having a light-permeable thick portion.

According to the light introduction device, the light is introduced into the medical conduit having the light-permeable thick portion, and therefore, the light can propagate to bacteria present in a substance contacting the medical conduit through the medical conduit. Thus, the bacteria present in the substance contacting the medical conduit can be deactivated by the light. In this manner, according to the light introduction device of the present invention, a sterilization effect against the substance contacting the medical conduit can be enhanced. Note that the medical conduit may be directly connected to the human body, or may be indirectly connected to the human body through, e.g., other medical conduits or an infusion needle.

Moreover, the above-described light introduction device preferably includes a light guide member surrounding the outer surface of the section of the medical conduit arranged outside the human body. One end portion of the light guide member facing one opening end side of the medical conduit is preferably diagonally inclined toward the medical conduit as extending toward one opening end side of the medical conduit, and the light entering from the other end side of the light guide member facing the other opening end side of the medical conduit is preferably entered to the medical conduit diagonally to a longitudinal direction of the medical conduit.

The light entered diagonally to the longitudinal direction of the medical conduit easily propagates in the longitudinal direction in the thick portion of the medical conduit as compared to light entered perpendicularly to the longitudinal direction of the medical conduit. Thus, the medical conduit itself serves as a waveguide, and the light can propagate to the substance contacting the medical conduit at a position apart from the light introduction device. Thus, according to the light introduction device, the sterilization effect against the substance contacting the medical conduit at the position apart from the light introduction device can be enhanced. For example, in a case where the medical conduit is arranged from the outside to the inside of the human body, when the opening end of the medical conduit on the inside of the human body is one opening end, bacteria interposed between the medical conduit and the human tissue can be, without passage through the human tissue, directly irradiated with the light through the medical conduit in a state in which the medical conduit is arranged in the human body. Thus, the bacteria interposed between the human tissue and a portion of the medical conduit surrounded by the human tissue can be deactivated such that the amount of the bacteria is decreased.

Further, the light guide member and the medical conduit preferably closely contact each other.

In this case, the amount of light introduced into the medical conduit through the light guide member can be increased as compared to a case where a clearance is formed between the light guide member and the medical conduit.

In addition, the above-described light introduction device preferably includes an optical fiber allowing entrance of the light from the outer surface of the section of the medical conduit arranged outside the human body. One end portion of the optical fiber preferably has an inclined surface inclined with respect to the center axis of the optical fiber, and is preferably arranged closer to the medical conduit as extending toward one opening end side of the medical conduit. The inclined surface preferably faces an outer surface of the medical conduit.

In this case, the amount of light entered to the medical conduit from the outer surface of the medical conduit can be increased as compared to an optical fiber configured such that an end surface of the optical fiber is arranged as described above perpendicularly to the center axis. Moreover, the direction of the light entered to the medical conduit is inclined with respect to the longitudinal direction of the medical conduit so that the inclined surface can easily contact the medical conduit. Thus, a loss of the light emitted from the optical fiber can be reduced, and such light can enter the medical conduit. As described above, the direction of the light entered to the medical conduit is inclined with respect to the longitudinal direction of the medical conduit. Thus, the light can propagate to the substance contacting the medical conduit at the position apart from the light introduction device as described above, and the sterilization effect against the substance contacting the medical conduit at the position apart from the light introduction device can be enhanced.

In this case, the angle between a line perpendicular to the inclined surface and the center axis of the optical fiber is preferably equal to or greater than 46 degrees, and the outer surface of the medical conduit and the inclined surface are preferably parallel to each other.

Generally, the refractive index of the optical fiber is higher than the refractive index of the medical conduit. Thus, according to the above-described configuration, the entrance angle of light entered to the outer surface of the medical conduit from the inclined surface of the optical fiber can be equal to or greater than 46 degrees. Thus, the amount of total reflection of the light entered from the outer surface to the thick portion of the medical conduit on an inner surface of the medical conduit can be increased as compared to a case where the entrance angle of light entered to the outer surface of the medical conduit is less than 46 degrees. Thus, the light can easily propagate, through the medical conduit, to the substance contacting the medical conduit at the position apart from the light introduction device.

Moreover, the light introduction device preferably further includes a fixing member configured to fix multiple optical fibers. The fixing member preferably has an arrangement surface arranged on the outer surface of the medical conduit, and preferably covers one end portion of each optical fiber such that an inclined surface of each optical fiber is exposed from an arrangement surface side.

In this case, the arrangement surface side of the fixing member fixing the multiple optical fibers is defined as an emission side of the light propagating in each optical fiber. Thus, the inclined surfaces of the optical fibers can easily face the outer surface of the medical conduit without separation of the multiple optical fibers.

Further, the fixing member preferably has flexibility, and the inclined surfaces of the multiple optical fibers are preferably arranged at intervals in one direction.

In this case, in a state in which the light is dispersed in a predetermined direction, the light can enter the medical conduit, and concentration of the light entering the medical conduit can be reduced. Moreover, the fixing member has the flexibility. Thus, the direction of arrangement of the optical fibers is coincident with a circumferential direction of the outer surface of the medical conduit so that the ultraviolet light can be introduced into the medical conduit from the circumferential direction. Thus, non-uniform introduction of the light in the circumferential direction of the medical conduit can be reduced. For example, in a case where the medical conduit is arranged from the outside to the inside of the human body, the light can be easily released from the circumferential direction of the medical conduit to the human tissue through the medical conduit, and as a result, a light irradiation region for the human tissue can be more expanded. Moreover, in a case where liquid is, for example, introduced into the inner cavity of the medical conduit, the liquid can be irradiated with the light from the circumferential direction of the medical conduit. Thus, the sterilization effect can be more enhanced.

In addition, the inclined surface of each optical fiber preferably closely contacts the outer surface of the medical conduit.

In this case, the loss of the light emitted from the optical fiber can be reduced, and the light can enter the medical conduit.

Moreover, the light preferably has a peak wavelength in a wavelength band of 270 nm to 340 nm.

In this case, the bacteria interposed between the medical conduit and the human tissue can be irradiated with light having a wavelength with a high sterilization ability, or the bacteria present in the liquid introduced into the medical conduit can be irradiated with such light. Thus, the sterilization effect can be more enhanced.

Further, the sterilization system of the present invention is characterized by including a medical conduit including an inner cavity allowing passage of at least one of a substance introduced into a human body or a substance discharged from the human body and including a light-permeable thick portion, and any of the above-described light introduction devices.

According to this sterilization system, the light can be introduced from the light introduction device into the medical conduit, and can propagate to the bacteria present in the substance contacting the medical conduit to deactivate the bacteria. Thus, according to the sterilization system of the present invention, the sterilization effect against the substance contacting the medical conduit can be enhanced.

In addition, liquid is preferably introduced into the inner cavity of the medical conduit.

The liquid introduced into the inner cavity of the medical conduit is the substance contacting an inner wall of the medical conduit, and the light can propagate in the substance through the medical conduit. Thus, bacteria present in the liquid can be deactivated such that the amount of the bacteria is decreased. When such liquid is the liquid introduced into the human body, the liquid with a reduced bacterium amount can be introduced into the human body.

Moreover, the medical conduit is preferably arranged from the outside to the inside of the human body.

The medical conduit itself having the light-permeable thick portion can serve as the waveguide. Thus, in a state in which part of the medical conduit is arranged in the human body, the bacteria interposed between the medical conduit and the human tissue can be irradiated with the light through the medical conduit. Thus, as compared to a case where the light is irradiated from the outside of the human body by way of the human tissue, the bacteria interposed between the human tissue and the medical conduit portion surrounded by the human tissue can be deactivated such that the amount of the bacteria is decreased. Note that in the case of introducing the liquid into the inner cavity of the medical conduit, the liquid can also serve as part of the waveguide.

In this case, at least part of the section of the medical conduit surrounded by the human tissue is preferably configured such that a light transmission for a wavelength band of 270 nm to 340 nm per millimeter in length is equal to or greater than 95%.

In the medical conduit, the light is easily released from the section where the light transmission for the wavelength band of 270 nm to 340 nm per millimeter in length is equal to or greater than 95% to the human tissue. Thus, the sterilization effect against the bacteria interposed between the human tissue and the conduit portion surrounded by the human tissue can be much more enhanced.

Further, the medical conduit preferably includes a first zone including the section surrounded by the human tissue, and a second zone on the opposite side of the first zone from the outside of the human body. The first zone is preferably configured such that the light transmission for the wavelength band of 270 nm to 340 nm per millimeter in length is equal to or greater than 95%. The second zone is preferably configured such that the light transmission for the wavelength band of 270 nm to 340 nm per millimeter in length is less than 95%.

In this case, the light transmitted through the first zone is attenuated in the second zone, and therefore, as compared to a case where the entirety of the medical conduit is formed by the first zone, emission of the light from, e.g., the end portion of the medical conduit can be reduced. Thus, unintended irradiation of the human tissue with the light in the wavelength band of 270 nm to 340 nm can be reduced, and influence of the light on the human tissue can be reduced.

In addition, the first zone preferably includes at least part of the section arranged outside the human body.

Since the first zone includes the section arranged outside the human body, the amount of attenuation of the light, which is introduced from the outside of the human body and propagates to the section surrounded by the human tissue, in the wavelength band of 270 nm to 340 nm can be reduced. Thus, the sterilization effect against the bacteria interposed between the human tissue and the conduit portion surrounded by the human tissue can be much more enhanced without the need for excessively increasing the intensity of light having the wavelength band of 270 nm to 340 nm and needing to propagate in the medical conduit.

Moreover, a recessed-raised portion is preferably provided at part of a surface of the section of the medical conduit surrounded by the human tissue.

In this case, as compared to the case of providing no recessed-raised portion, the amount of light emitted from the portion provided with the recessed-raised portion can be increased. Thus, the amount of light released from the medical conduit to the human tissue can be adjusted by the recessed-raised portion.

Further, a height difference at the recessed-raised portion preferably increases with a distance from the opening end of the medical conduit on the outside of the human body.

In this case, a decrease in the amount of light emitted from the portion provided with the recessed-raised portion with a distance from the human body outer side of the medical conduit is suppressed. Non-uniformity in the intensity of light emitted from the portion provided with the recessed-raised portion of the medical conduit along the longitudinal direction of the medical conduit can be reduced.

In addition, at part of the surface of the section of the medical conduit surrounded by the human tissue, multiple reflectors dispersed on the surface are preferably provided.

In this case, a predetermined amount of light released from the section surrounded by the human tissue to the human tissue can be ensured while the light can easily propagate to a lumen side of the human body.

Moreover, the percentage of the reflectors per unit area of the medical conduit preferably decreases with a distance from the opening end of the medical conduit on the outside of the human body.

In this case, a decrease in the amount of light emitted from the portion provided with the reflectors with a distance from the human body outer side of the medical conduit is suppressed. Thus, non-uniformity in the intensity of light emitted from the portion provided with the reflectors of the medical conduit along the longitudinal direction of the medical conduit can be reduced.

### Brief Description of Drawings

FIG. 1 is a view of an outline configuration of a sterilization system in a first embodiment.
FIG. 2 is a view of the outline of a peritoneal dialysis catheter in a state in which the catheter is not placed in a human body.
FIG. 3 is a view of the outline of a light introduction device in a closed state.
FIG. 4 is a view of the outline of the light introduction device in an open state.
FIG. 5 is a sectional view of the state of attachment of the light introduction device to the catheter along an X-X section of FIG. 3.
FIG. 6 is a sectional view of a light propagation state from the light introduction device.
FIG. 7 is a view of a configuration of a light introduction device provided at a sterilization system in a second embodiment from the same view point as that of FIG. 5.
FIG. 8 is a view of an outline configuration of a sterilization system in a third embodiment.
FIG. 9 is a view of an outline configuration of a light introduction device provided at a sterilization system in a fourth embodiment.
FIG. 10A - FIG. 10C illustrate views of the state of manufacturing of the light introduction device.
FIG. 11 is a view of the state of attachment of the light introduction device.
FIG. 12 is a sectional view of a light propagation state.
FIG. 13 is a view of an outline configuration of a dialysis device including a sterilization system in a fifth embodiment.
FIG. 14 is a view of the state of light propagation from a light introduction device in the fifth embodiment as in FIG. 6.
FIG. 15 is a view of an outline configuration of an infusion device including a sterilization system in a sixth embodiment.
FIG. 16 is an enlarged view of a portion surrounded by a chain line of Fig. 2, illustrating a state in which a surface of a catheter is processed.

### Description of Embodiments

Hereinafter, embodiments for carrying out the present invention and variations thereof will be described by way of example with reference to the attached drawings. The embodiments and the variations described below by way of example are for the sake of easy understanding of the present invention, and are not intended to interpret the present invention in a limited manner. Changes and modifications can be made to the present invention without departing from the gist thereof.

### (1) First Embodiment

### <Configuration of Sterilization System>

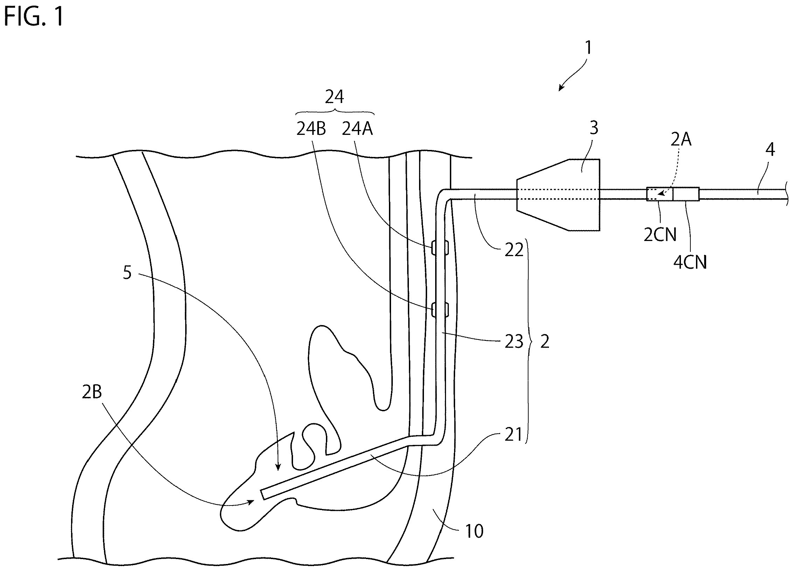

FIG. 1 is a view of an outline configuration of a sterilization system in a first embodiment. As illustrated in FIG. 1, a sterilization system 1 of the present embodiment includes, as main components, a peritoneal dialysis catheter 2 and a light introduction device 3.

The catheter 2 is a medical conduit used as a flow path through which a dialysate solution is taken in or out of a peritoneal cavity, and is in such a tubular shape that a thick portion surrounds an inner cavity. In the present embodiment, the catheter 2 has an intracorporeal arrangement portion 21 as a section arranged in a human abdominal area from the outside to the inside of a human body and arranged in the peritoneal cavity as the inside of the human body, an extracorporeal arrangement portion 22 as a section arranged outside the human body, and a human tissue arrangement portion 23 as a section surrounded by a human tissue 10. Note that a portion of the human tissue 10 where the human tissue arrangement portion 23 is placed is sometimes called a "subcutaneous tunnel."

The light introduction device 3 is provided at a middle section of the extracorporeal arrangement portion 22. An opening end 2A of the extracorporeal arrangement portion 22 is covered with a connector 2CN, and communicates with an opening end of an external tube 4 on one end side through an external connector 4CN connected to the connector 2CN. Note that in a state in which the connector 2CN of the catheter 2 and the external connector 4CN of the external tube 4 are connected to each other, the dialysate solution can be taken in or out of the peritoneal cavity through the catheter 2 and the external tube 4.

Cuffs 24 are provided at the human tissue arrangement portion 23. The cuff 24 is a fibrous member provided on a surface of the human tissue arrangement portion 23, and is fixed to the human tissue 10 by adhesion to the human tissue 10 to reduce unintended detachment of the catheter 2. The cuffs 24 of the present embodiment include an external cuff 24A and an internal cuff 24B. The external cuff 24A is positioned closer to the opening end 2A of the catheter 2 on a human body outer side, and the internal cuff 24B is positioned closer to an opening end 2B of the catheter 2 on a human body inner side. Note that the number of cuffs 24 may be one or three or more, and the cuffs 24 may be omitted.

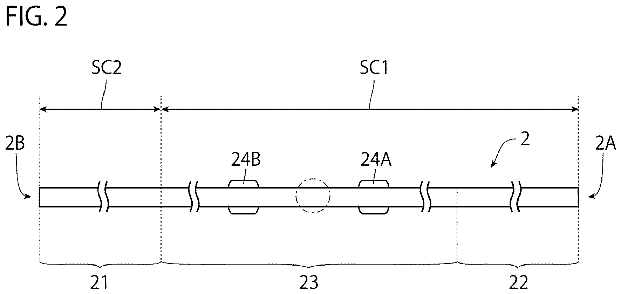

FIG. 2 is a view of the outline of the peritoneal dialysis catheter 2 not placed in the human body. As illustrated in FIG. 2, the catheter 2 of the present embodiment includes a first zone SC1 where a light transmission for a wavelength band of 270 nm to 340 nm per millimeter in length is equal to or higher than 95%, and a second zone SC2 where the light transmission for the wavelength band of 270 nm to 340 nm per millimeter in length is lower than 95%. Note that the light transmission for the wavelength band of 270 nm to 340 nm per millimeter in length means the percentage of light, having the wavelength band of 270 nm to 340 nm, passing through a portion from a certain spot of the catheter 2 to a point apart from the certain spot by 1 mm in a longitudinal direction of the catheter 2. Moreover, light included in the wavelength band of 270 nm to 340 nm will be hereinafter referred to as "ultraviolet light."

In the case of the present embodiment, the extracorporeal arrangement portion 22 and the human tissue arrangement portion 23 of the catheter 2 are taken as the first zone SC1, and the intracorporeal arrangement portion 21 of the catheter 2 is taken as the second zone SC2. That is, a zone from the opening end 2A of the catheter 2 on the human body outer side to a boundary position between the human tissue arrangement portion 23 and the intracorporeal arrangement portion 21 is taken as the first zone SC1, and a zone from such a boundary position to the opening end 2B of the catheter 2 on the human body inner side is taken as the second zone SC2. Thus, in a case where the catheter 2 is placed in the human body as illustrated in FIG. 1, the second zone SC2 is positioned on the opposite side of the first zone SC1 from the human body outer side. The catheter 2 is made of a material allowing transmission of the ultraviolet light, and such a material of the catheter 2 includes, for example, silicone resin. The ultraviolet transmission of the silicone resin varies depending on the type of such resin, and the transmission varies according to a material composition, a molecular structural skeleton, an additive agent, and the type and amount of filler. Thus, the material is selected as necessary so that the transmissions of the first zone SC1 and the second zone SC2 can be differentiated from each other. Moreover, in the case of obtaining a high transmission in an ultraviolet range, it is effective to decrease an additive agent having an ultraviolet absorption ability or not to use such an agent.

The length of the catheter 2 is such a length that the opening end 2B of the catheter 2 on the human body inner side is arranged in a recessed portion 5 (FIG. 1) of the peritoneal cavity. Note that the recessed portion 5 of the peritonea is sometimes called a "Douglas' pouch" as a dent of the peritonea. In the case of a male, the recessed portion 5 is positioned between the bladder and the rectum. In the case of a female, the recessed portion 5 is positioned between the uterus and the rectum. The inner diameter of the catheter 2 is within a range of equal to or greater than 2.0 mm and equal to or less than 4.0 mm, and the thickness of the catheter 2 is within a range of equal to or greater than 0.5 mm and equal to or less than 1.5 mm. Note that the thickness of the catheter 2 is a difference between the outer diameter and the inner diameter of the catheter 2.

The light introduction device 3 is a device configured to introduce the ultraviolet light from the extracorporeal arrangement portion 22 of the catheter 2, and in the present embodiment, is detachable from the extracorporeal arrangement portion 22 of the catheter 2.

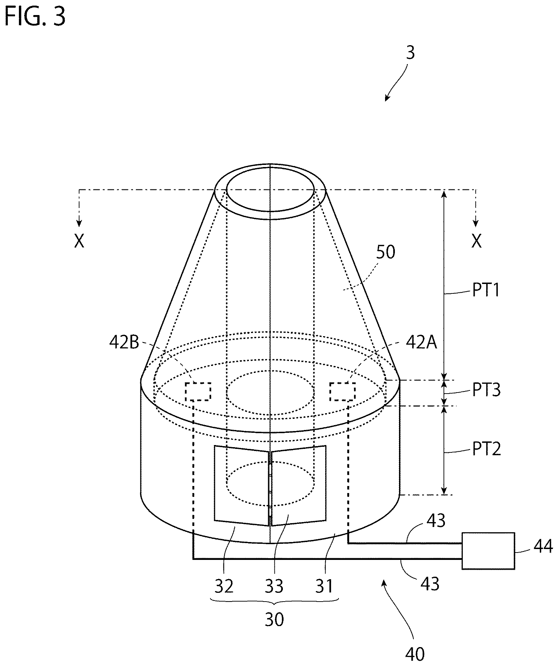

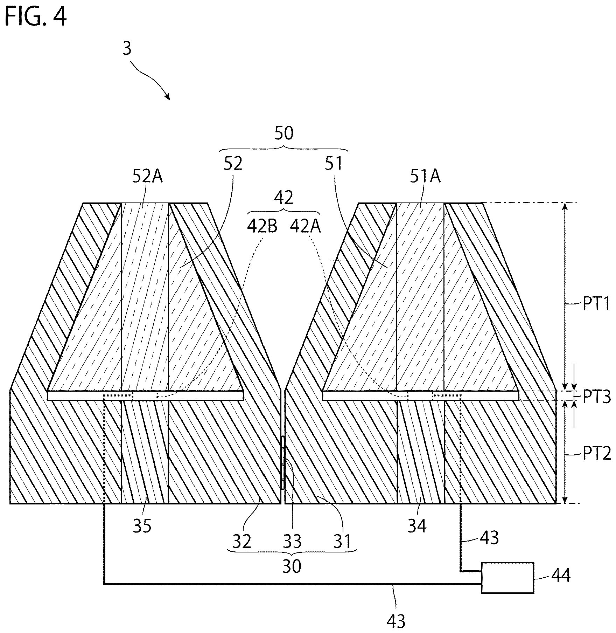

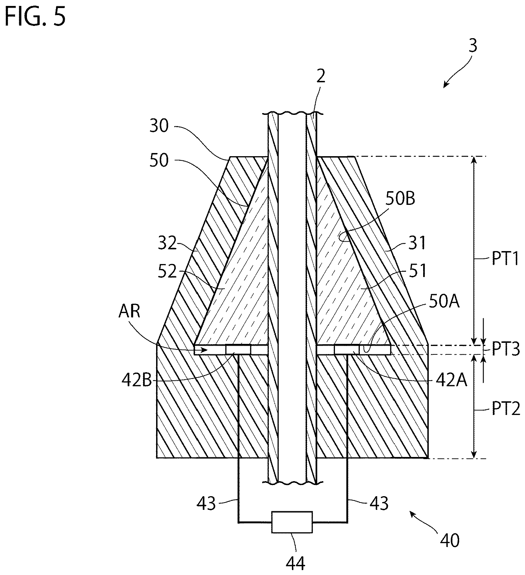

FIG. 3 is a view of the outline of the light introduction device in a closed state, FIG. 4 is a view of the outline of the light introduction device in an open state, and FIG. 5 is a sectional view of the state of attachment of the light introduction device to the catheter along an X-X section of FIG. 3. As illustrated in FIGS. 3 to 5, the light introduction device 3 of the present embodiment includes, as main components, a housing 30, a light emitter 40, and a light guide member 50.

The housing 30 is a tubular member surrounding the extracorporeal arrangement portion 22 of the catheter 2. The housing 30 may be made of a substantially ultraviolet-impermeable material.

In the case of the present embodiment, the housing 30 includes a first tube portion PT1, a second tube portion PT2, and a third tube portion PT3. The first tube portion PT1 is a frusto-conical member whose outer and inner diameters are narrowed with a distance from the second tube portion PT2, and the light guide member 50 is attached to the inside of such a tube.

The second tube portion PT2 is a circular tube-shaped member having an inner diameter substantially equal to the outer diameter of the catheter 2, and can fix part of the extracorporeal arrangement portion 22 of the catheter 2.

The third tube portion PT3 is a circular tube-shaped member having an inner diameter greater than that of the second tube portion PT2, and the light guide member 50 attached to the first tube portion PT1 and an end surface of the second tube portion PT2 face each other through a space AR inside such a tube.

The housing 30 of the present embodiment as described above is openable by a first half body 31, a second half body 32 having the substantially same shape as that of the first half body 31, and a hinge 33 configured to open/close the first half body 31 and the second half body 32. The first half body 31 and the second half body 32 are closed through the hinge 33 such that an inner wall surface of the first half body 31 and an inner wall surface of the second half body 32 face each other, and such a closed state is maintained by a not-shown stopper.

A groove portion 34 is provided at the inner wall surface of the first half body 31 at the second tube portion PT2, and a groove portion 35 is provided at the inner wall surface of the second half body 32 at the second tube portion PT2. In a case where the first half body 31 and the second half body 32 are brought into the closed state in such a manner that the pair of groove portions 34, 35 enters part of the extracorporeal arrangement portion 22, the extracorporeal arrangement portion 22 is fixed by the second tube portion PT2, and the light introduction device 3 is fixed to the catheter 2. That is, in the present embodiment, the light introduction device is detachably fixed to the catheter 2 by sandwiching of the catheter 2.

The light emitter 40 is configured to emit light having a peak wavelength in the wavelength band of 270 nm to 340 nm, and includes LEDs 42, electric wires 43, and a power source 44. The LEDs 42 are, in the housing 30, arranged on the end surface of the second tube portion PT2 facing the light guide member 50 attached to the first tube portion PT1, and the power source 44 is arranged outside the housing 30. The electric wires 43 connect, through a wall of the second tube portion PT2, the LEDs 42 arranged inside the housing 30 and the power source 44 arranged outside the housing 30, for example. The light emitter 40 turns on the LEDs 42 based on power supplied from the power source 44 through the electric wires 43, and emits the ultraviolet light to the light guide member 50.

In the case of the present embodiment, the LEDs 42 have, as illustrated in FIG. 4, a first LED 42A arranged on the end surface of the second tube portion PT2 at the first half body 31, and a second LED 42B arranged on the end surface of the second tube portion PT2 at the second half body 32. In FIG. 4, the first LED 42A is arranged on a far side with respect to the groove portion 34 in a direction perpendicular to the plane of paper, and therefore, is indicated by a dashed line. The second LED 42B is also arranged on the far side with respect to the groove portion 35 in the direction perpendicular to the plane of paper, and therefore, is indicated by a dashed line. Note that the LEDs 42 are two LEDs including the first LED 42A and the second LED 42B in the present embodiment, but may include a single LED or three or more LEDs. Moreover, the LEDs 42 are preferably arranged such that an incident surface of the light guide member 50 is uniformly irradiated with light.

The light guide member 50 is a tubular member surrounding a side surface of part of the extracorporeal arrangement portion 22 of the catheter 2. The material of the light guide member 50 includes, for example, quartz.

In the case of the present embodiment, the light guide member 50 includes, as illustrated in FIG. 4, a first light guide half body 51 fixed to an inner wall of the first half body 31, and a second light guide half body 52 fixed to an inner wall of the second half body 32. A groove portion 51A is provided on the opposite surface side of the surface side contacting the inner wall of the first half body 31 at the first light guide half body 51, and a groove portion 52A is provided on the opposite surface side of the surface side contacting the inner wall of the second half body 32 at the second light guide half body 52.

In a case where the first half body 31 and the second half body 32 are brought into the closed state in such a manner that part of the extracorporeal arrangement portion 22 of the catheter 2 is sandwiched by the pair of groove portions 51A, 52A, the first light guide half body 51 fixed to the first half body 31 and the second light guide half body 52 fixed to the second half body 32 form the light guide member 50 as illustrated in FIG. 5. In this case, the side surface of part of the extracorporeal arrangement portion 22 of the catheter 2 is surrounded by the light guide member 50, and the light guide member 50 and the catheter 2 closely contact each other.

Of the light guide member 50 surrounding part of the extracorporeal arrangement portion 22 of the catheter 2 as described above, an entrance-side end portion 50A facing an opening end 2A side of the extracorporeal arrangement portion 22 of the catheter 2 contacts light emission surfaces of the first LED 42A and the second LED 42B, and light emitted from the first LED 42A and the second LED 42B enters the entrance-side end portion 50A. Of the light guide member 50, an emitting-side end portion 50B facing an opening end 2B side of the catheter 2 on the human body inner side is, on the other hand, diagonally inclined toward the catheter 2 as extending toward the opening end 2B of the catheter 2 on the human body inner side. That is, when the opening end 2B of the catheter 2 on the human body inner side is one opening end, the opening end 2A of the extracorporeal arrangement portion 22 is the other opening end, the emitting-side end portion 50B of the light guide member 50 is one end portion, and the entrance-side end portion 50A is the other end portion, one end portion of the light guide member 50 facing one opening end side of the catheter 2 is diagonally inclined toward the catheter 2 as extending toward one opening end side of the catheter 2, and light is entered from the other end side of the light guide member 50 facing the other opening end side of the catheter 2.

### <Sterilization Method by Sterilization System>

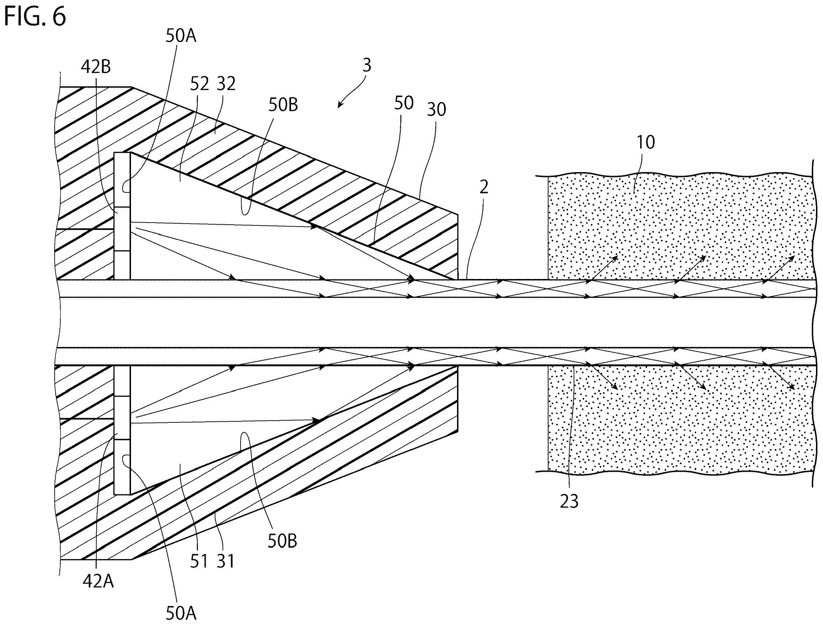

Next, a sterilization method by the sterilization system 1 of the present embodiment will be described with reference to FIG. 6. FIG. 6 is a sectional view of a light propagation state. Note that in FIG. 6, hatching of the section of the catheter 2 is omitted for the sake of easy understanding of the light propagation state.

In the case of sterilizing, e.g., the catheter 2 placed in the human body, part of the extracorporeal arrangement portion 22 of the catheter 2 is sandwiched by the groove portions 34, 35 of the second tube portion PT2, the groove portion 51A of the first light guide half body 51, and the groove portion 52A of the second light guide half body 52. Thereafter, the first half body 31 and the second half body 32 are closed, and in such a closed state, the first LED 42A and the second LED 42B are turned on.

As illustrated in FIG. 6, in the case of turning on the first LED 42A and the second LED 42B, the ultraviolet light is emitted from the first LED 42A and the second LED 42B. Such ultraviolet light enters the light guide member 50 through the entrance-side end portion 50A of the light guide member 50 facing the LEDs 42, and propagates toward the emitting-side end portion 50B of the light guide member 50.

As described above, the emitting-side end portion 50B of the light guide member 50 is diagonally inclined toward the catheter 2 as extending toward the opening end 2B of the catheter 2 on the human body inner side. Thus, large part of light propagating from the entrance-side end portion 50A of the light guide member 50 to the emitting-side end portion 50B is reflected on the emitting-side end portion 50B. The light reflected on the emitting-side end portion 50B propagates toward the catheter 2 in a state diagonally to the longitudinal direction of the catheter 2, and enters the thick portion of the catheter 2. Note that a reflector configured to reflect incident light from the entrance-side end portion 50A toward the catheter 2 may be provided on the surface of the emitting-side end portion 50B. A metal thin film made of aluminum or silver can be utilized as the reflector.

As described above, in the present embodiment, the transmission for the ultraviolet light per millimeter in length at the extracorporeal arrangement portion 22 and the human tissue arrangement portion 23 of the catheter 2 is equal to or higher than 95%. Thus, large part of light having entered the thick portion of the catheter 2 can propagate, using the catheter 2 as a waveguide, toward the opening end 2B of the catheter 2 on the human body inner side in the thick portion with substantially no attenuation.

The catheter 2 contacts atmospheric air at the extracorporeal arrangement portion 22, and therefore, light propagating in the thick portion of the extracorporeal arrangement portion 22 propagates with leakage to the outside of the catheter 2 being reduced in a total reflection mode. Moreover, the human tissue arrangement portion 23 of the catheter 2 is surrounded in contact with the human tissue, and a refractive index difference between the human tissue arrangement portion 23 and the human tissue 10 is smaller than a refractive index difference between the extracorporeal arrangement portion 22 and the atmospheric air. Thus, part of light reaching the human tissue arrangement portion 23 is released from the human tissue arrangement portion 23 to the human tissue 10. Thus, in a case where bacteria is interposed between the human tissue arrangement portion 23 of the catheter 2 and the human tissue 10, the bacteria might be directly irradiated with the ultraviolet light. Note that for light propagation in the thick portion of the catheter 2 as described above, light is preferably emitted from the LEDs 42 before the inner cavity of the catheter 2 is filled with the dialysate solution. Note that light may be emitted from the LEDs 42 in a state in which the inner cavity of the catheter 2 is filled with the dialysate solution.

In a case where a light (a light of about 1 mW/cm²) of about 1 mW per square area of 1 cm in length and 1 cm in width is released from the human tissue arrangement portion 23, if the peak wavelength emitted from the LED 42 is 265 nm, the rate of sterilization by light is 99% in an irradiation time of about four seconds. Moreover, if the peak wavelength emitted from the LED 42 is 320 nm, the rate of sterilization by light is 99% in an irradiation time of about 50 minutes.

Note that as described above, the transmission for the ultraviolet light per millimeter in length at the intracorporeal arrangement portion 21 of the catheter 2 is lower than 95%, and the length of the intracorporeal arrangement portion 21 is greater than the length of the extracorporeal arrangement portion 22. Thus, large part of light propagating in the intracorporeal arrangement portion 21 through the human tissue arrangement portion 23 of the catheter 2 is attenuated, and leakage of light from the opening end 2B of the catheter 2 on the human body inner side is reduced.

### <Summary>

As described above, the light introduction device 3 of the present embodiment introduces light through part of an outer surface of a section of the catheter 2 arranged outside the human body, the catheter 2 being the medical conduit having the inner cavity for passage of at least one of a substance introduced into the human body or a substance discharged from the human body and having the light-permeable thick portion. Moreover, the sterilization system 1 using the light introduction device 3 includes the catheter 2 and the light introduction device 3.

According to the sterilization system 1 and the light introduction device 3 as described above, light is introduced into the light-permeable medical conduit so that the light can propagate to even bacteria present in a substance contacting the catheter 2 through the catheter 2 as the medical conduit. Thus, the bacteria present in the substance contacting the catheter 2 can be deactivated by the light. In this manner, according to the sterilization system 1 and the light introduction device 3 of the present invention, a sterilization effect against the substance contacting the medical conduit can be enhanced.

Moreover, the light introduction device 3 of the present embodiment includes the light guide member 50 surrounding the side surface of the extracorporeal arrangement portion 22, and the light guide member 50 is detachable from the catheter 2. Moreover, the emitting-side end portion 50B facing the human body inner side of the catheter 2 is diagonally inclined toward the catheter 2 as extending toward the human body inner side of the catheter 2, and causes light entered from an entrance-side end portion 50A side facing the human body outer side of the catheter 2 to enter diagonally to the longitudinal direction of the catheter 2.

Thus, even in a state in which the catheter 2 is already arranged in the human body, more light can be introduced into the catheter 2. Moreover, even if the light introduction device 3 is unexpectedly damaged, the light introduction device 3 can be replaced with the catheter 2 remaining in the human body.

Moreover, in the light introduction device 3 of the present embodiment, the light guide member 50 and the catheter 2 closely contact each other. Thus, as compared to a case where a clearance is formed between the light guide member 50 and the catheter 2, the amount of light introduced into the catheter 2 from the LEDs 42 through the light guide member 50 can be increased.

Further, the light introduction device 3 of the present embodiment causes the light having the peak wavelength in the wavelength band of 270 nm to 340 nm to be entered to the catheter 2.

Thus, the bacteria interposed between the catheter 2 and the human tissue 10 can be, without passage through the human tissue 10, directly irradiated with light having a wavelength with a high sterilization ability against the bacteria through the catheter 2.

In addition, in the sterilization system 1 of the present embodiment, the human tissue arrangement portion 23 of the catheter 2 is configured such that the light transmission for the wavelength band of 270 nm to 340 nm per millimeter in length is equal to or higher than 95%.

Thus, in the catheter 2, light is easily released toward the human tissue 10 from the section where the light transmission for the wavelength band of 270 nm to 340 nm per millimeter in length is equal to or higher than 95%. Thus, the sterilization effect against the bacteria interposed between the human tissue 10 and a conduit portion surrounded by the human tissue 10 can be further enhanced.

Moreover, the catheter 2 of the present embodiment includes the first zone SC1 and the second zone SC2 on the opposite side of the first zone SC1 from the human body outer side. The first zone SC1 is configured such that the light transmission for the wavelength band of 270 nm to 340 nm per millimeter in length is equal to or higher than 95%, and the second zone SC2 is configured such that the light transmission for the wavelength band of 270 nm to 340 nm per millimeter in length is lower than 95%.

Thus, as compared to a case where the entirety of the catheter 2 is formed by the first zone SC1, unintended irradiation of the human tissue 10 with the light in the wavelength band of 270 nm to 340 nm from the catheter 2 can be reduced, and influence of light on the human tissue 10 can be reduced.

Moreover, the first zone SC1 of the catheter 2 of the present embodiment includes the intracorporeal arrangement portion 21. Thus, the amount of attenuation of the light in the wavelength band of 270 nm to 340 nm can be reduced, the light being introduced from the outside of the human body and propagating to the human tissue arrangement portion 23. Thus, the sterilization effect against the bacteria interposed between the human tissue 10 and the human tissue arrangement portion 23 can be further enhanced without the need for excessively increasing the intensity of the light having the wavelength band of 270 nm to 340 nm and needing to propagate in the catheter 2.

### (2) Second Embodiment

Next, a second embodiment will be described. Note that the same reference numerals are used to represent configurations similar to those described in the first embodiment, and unless otherwise described, overlapping description will be described.

### <Configuration of Sterilization System>

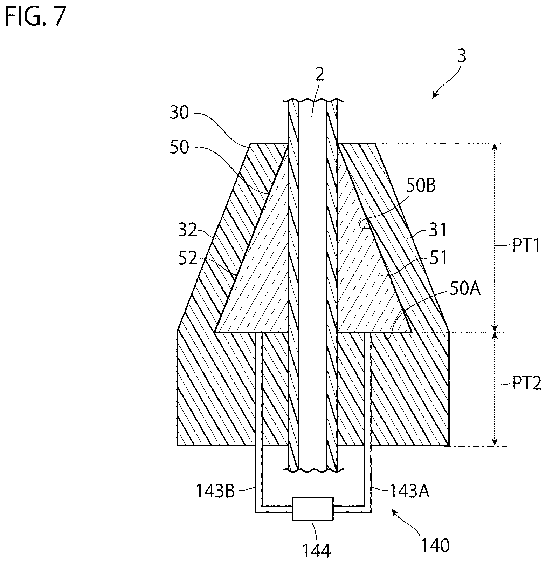

FIG. 7 is a view of a configuration of a light introduction device provided at a sterilization system in the second embodiment from the same view point as that of FIG. 5. As illustrated in FIG. 7, in the sterilization system of the present embodiment, a third tube portion PT3 of a housing 30 is omitted, and a first tube portion PT1 and a second tube portion PT2 form the housing 30. Moreover, the sterilization system of the present embodiment is different from that of the above-described first embodiment in that a light emitter 140 having a different configuration from that of the light emitter 40 is employed instead of the light emitter 40 of the first embodiment.

The light emitter 140 includes two optical fibers 143A, 143B and a light source 144. One end portion of each of the optical fibers 143A, 143B is optically connected to the light source 144. Moreover, the other end portion of each of the optical fibers 143A, 143B is inserted into a through-hole provided along a longitudinal direction of the second tube portion PT2, and is fixed to the second tube portion PT2. An end surface on an end side of each of the optical fibers 143A, 143B fixed to the second tube portion PT2 contacts an entrance-side end portion 50A of a light guide member 50.

Note that two optical fibers 143A, 143B are employed in the present embodiment, but may be one or three or more. Moreover, the end surfaces of the optical fibers 143A, 143B on the side fixed to the second tube portion PT2 may be apart from an end surface of the entrance-side end portion 50A of the light guide member 50.

The light emitter 140 as described above irradiates the entrance-side end portion 50A of the light guide member 50 with ultraviolet light generated by the light source 144 through the optical fibers 143A, 143B.

Light entering the entrance-side end portion 50A of the light guide member 50 from the light emitter 140 is, as in the case of the above-described first embodiment, reflected on an emitting-side end portion 50B of the light guide member 50, and enters a thick portion of a catheter 2. The light having entered the thick portion of the catheter 2 propagates, as in the case of the above-described first embodiment, in the catheter 2 as a waveguide, and is released from a human tissue arrangement portion 23 of the catheter 2 toward a human tissue 10.

Thus, as in the above-described first embodiment, the sterilization system of the present embodiment can directly irradiate, without passage through the human tissue 10, bacteria interposed between the catheter 2 and the human tissue 10 with the ultraviolet light. Thus, as compared to a case where ultraviolet light is irradiated from the outside of a human body, bacteria interposed between the human tissue 10 and a conduit portion surrounded by the human tissue 10 can be sterilized such that the amount of such bacteria is decreased.

### (3) Third Embodiment

Next, a third embodiment will be described. Note that the same reference numerals are used to represent configurations similar to those described in the first embodiment, and unless otherwise described, overlapping description will be omitted.

### <Configuration of Sterilization System>

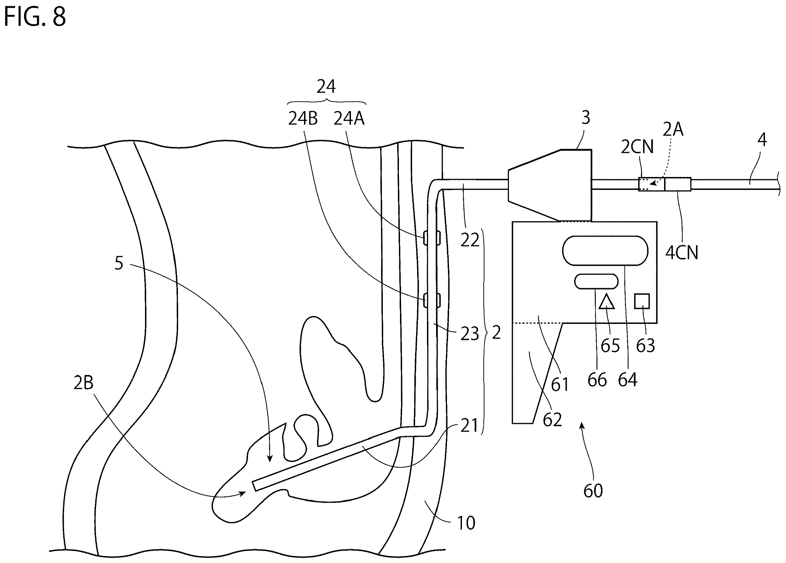

FIG. 8 is a view of an outline configuration of a sterilization system in the third embodiment. As illustrated in FIG. 8, the sterilization system 1 of the present embodiment is different from that of the above-described first embodiment in that a control device 60 configured to control a light introduction device 3 is newly provided.

The control device 60 has a housing 61 integrally molded with a housing 30 in the light introduction device 3, and a gripping portion 62 molded integrally with the housing 61. Note that the housing 61 may be formed integrally with a first half body 31 of the housing 30 or a second half body 32 of the housing 30.

At a surface of the housing 61, a power button 63 configured to switch ON/OFF of power and a time display 64 configured to display time after LEDs 42A, 42B has started lighting are provided. Moreover, at the surface of the housing 61, a connection switch button 65 configured to switch connection with the power source 44 between the first LED 42A and the second LED 42B and a wavelength display 66 configured to display a current peak wavelength are provided. Further, the power source 44 and electric wires 43 of a light emitter 40 are provided inside the housing 61. Note that in the present embodiment, the peak wavelength of light emitted from the first LED 42A and the peak wavelength of light emitted from the second LED 42B are different from each other.

In a case where the power button 63 is switched from OFF to ON, the control device 60 takes such a switch point as a trigger to supply power from the power source 44 to, e.g., the first LED 42A until the power button 63 is switched from ON to OFF, thereby continuing ON of the first LED 42A. In addition, the control device 60 measures time after the point of switching the power button 63 from OFF to ON, and displays, as irradiation time, a measurement result on the time display 64 until the power button 63 is switched from ON to OFF.

Thus, the sterilization system 1 of the present embodiment can intuitively provide the irradiation time to a user, and can irradiate bacteria interposed between a catheter 2 and a human tissue 10 with ultraviolet light for such proper irradiation time that influence on the human tissue 10 does not reach equal to or greater than a certain degree.

Moreover, in a case where the connection switch button 65 is switched from the first LED 42A to the second LED 42B, the control device 60 takes such a switch point as a trigger to disconnect the power source 44 and the first LED 42A from each other and connect the power source 44 and the second LED 42B to each other. At this point, on the wavelength display 66, the control device 60 displays, as the current peak wavelength, the peak wavelength of the second LED 42B instead of the peak wavelength of the first LED 42A displayed on the wavelength display 66. On the other hand, in a case where the connection switch button 65 is switched from the second LED 42B to the first LED 42A, the control device 60 takes such a switch point as a trigger to disconnect the power source 44 and the second LED 42B from each other and connect the power source 44 and the first LED 42A to each other. At this point, on the wavelength display 66, the control device 60 displays, as the current peak wavelength, the peak wavelength of the first LED 42A instead of the peak wavelength of the second LED 42B displayed on the wavelength display 66.

As the wavelength of light released from the catheter 2 to the human tissue 10 becomes closer to 270 nm, a sterilization capability against the bacteria interposed between the catheter 2 and the human tissue 10 is improved. Meanwhile, the influence on the human tissue 10 increases. On the other hand, as the wavelength of light released from the catheter 2 to the human tissue 10 becomes closer to 340 nm, the sterilization capability against the bacteria interposed between the catheter 2 and the human tissue 10 is reduced. Meanwhile, the influence on the human tissue 10 decreases. Thus, the sterilization system 1 of the present embodiment can more improve the sterilization capability according to, e.g., ultraviolet absorption characteristics of the human tissue 10 surrounding the catheter 2 and the irradiation time of light for irradiation of the human tissue 10 while performing adjustment such that the influence on the human tissue 10 decreases. Note that the first LED 42A and the second LED 42B having different peak wavelengths are switched, but an LED whose peak wavelength is switchable may be used.

### (4) Fourth Embodiment

Next, a fourth embodiment will be described. Note that the same reference numerals are used to represent configurations similar to those described in the first embodiment, and unless otherwise described, overlapping description will be omitted.

### <Configuration of Sterilization System>

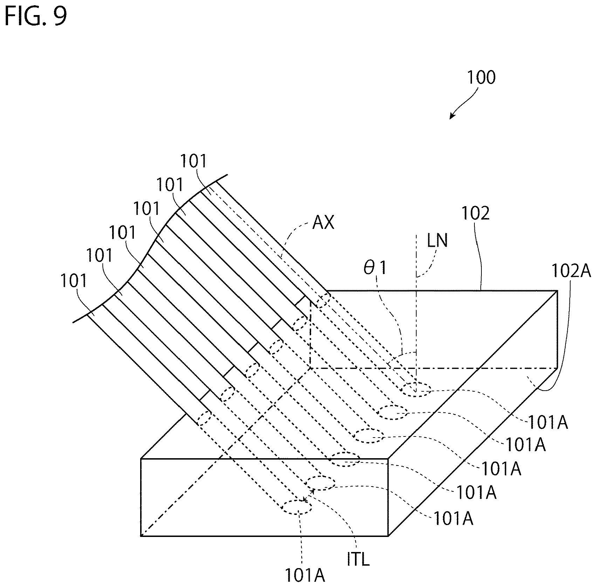

FIG. 9 is a view of an outline configuration of a light introduction device provided at a sterilization system in the fourth embodiment. As illustrated in FIG. 9, a light introduction device 100 of the present embodiment is a device configured to introduce ultraviolet light from an extracorporeal arrangement portion 22 of a catheter 2, and includes multiple optical fibers 101 and a fixing member 102 as main components.

The multiple optical fibers 101 are members in which light propagates, and each optical fiber 101 has a core and a clad covering the core. A difference between the refractive index of the core of each optical fiber 101 and the refractive index of the catheter 2 is about 3%. Such a refractive index difference is preferably equal to or lower than 3%. Moreover, one end portion of each optical fiber 101 has an inclined surface 101A inclined with respect to the center axis AX of the optical fiber. An angle θ1 between a line LN perpendicular to the inclined surface 101A and the center axis AX of the optical fiber is equal to or greater than 46 degrees. Note that the center axis AX of the optical fiber can be also understood as the optical axis of the optical fiber. The angle θ1 corresponds to the entrance angle of light propagating in the core of the optical fiber and entering an interface with the inclined surface 101A from the inclined surface 101A.

The fixing member 102 is a member configured to fix the multiple optical fibers 101, and has flexibility. The fixing member 102 has an arrangement surface 102A arranged on an outer surface of the catheter 2, and covers end portions of the multiple optical fibers 101 from an arrangement surface 102A side such that each of the inclined surfaces 101A of the multiple optical fibers 101 are exposed.

In the case of the present embodiment, the inclined surface 101A of each of the multiple optical fibers 101 is positioned on the substantially same plane as that of the arrangement surface 102A of the fixing member 102. Note that as long as the inclined surface 101A of each of the multiple optical fibers 101 is substantially parallel to the arrangement surface 102A of the fixing member 102, the inclined surface 101A is not necessarily positioned on the arrangement surface 102A of the fixing member 102. Note that in a state in which the arrangement surface 102A contacts the outer surface of the catheter 2, the inclined surface 101A of the optical fiber 101 is preferably positioned on the arrangement surface 102A of the fixing member 102 not to reflect light emitted from the inclined surface 101A on the outer surface of the catheter 2.

Moreover, in the case of the present embodiment, the inclined surfaces 101A of the multiple optical fibers 101 are arranged at intervals ITL in a predetermined direction. Note that as long as the inclined surfaces 101A of the multiple optical fibers 101 are arranged at the intervals ITL in the predetermined direction, a position relationship among the optical fibers 101 may be shifted to a certain degree. Moreover, various arrangement forms may be employed as arrangement of the inclined surface 101A of each of the multiple optical fibers 101. For example, the inclined surfaces 101A of the multiple optical fibers 101 may be arranged in two lines parallel to each other, or may be arranged in a grid pattern. Note that the inclined surfaces 101A are preferably arranged at the intervals ITL in the predetermined direction so that the inclined surface 101A of each of the multiple optical fibers 101 can be arranged along a circumferential direction of the outer surface of the catheter 2.

Note that end portions of the multiple optical fibers 101 on the opposite side of an inclined surface 101A side are connected to a not-shown light source unit, and the ultraviolet light is introduced from the light source unit to the core of each optical fiber 101. For example, in a case where the diameter of the core of the optical fiber 101 is 1 mm, an output of about 50 mW per optical fiber 101 can be obtained.

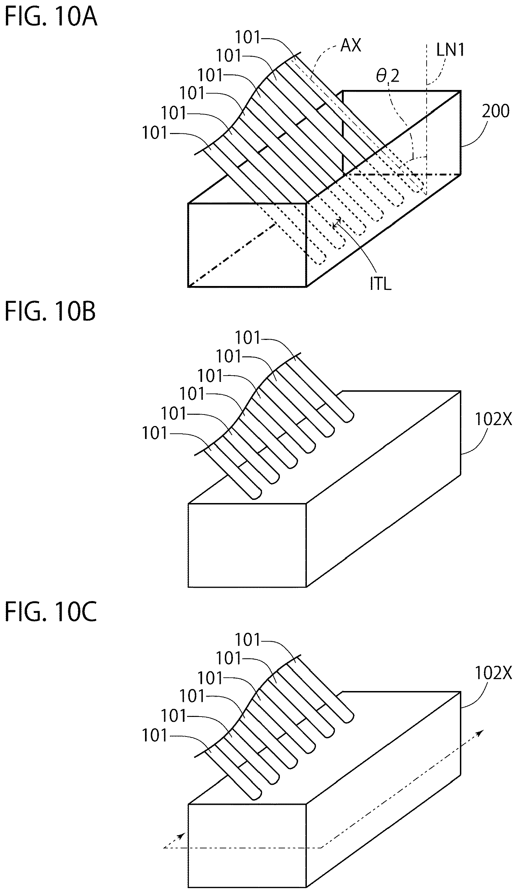

The above-described light introduction device 100 can be, for example, manufactured as follows. FIG. 10A - FIG. 10C illustrate views of the state of manufacturing of the light introduction device.

First, as illustrated in FIG. 10A, e.g., a box 200 having a rectangular parallelepiped space is prepared, and the multiple optical fibers 101 are, on one end side thereof, diagonally arranged at the intervals ITL in the box 200. An angle θ2 between a line LN1 perpendicular to a bottom surface of the box 200 and the center axis AX of the optical fiber corresponds to the angle θ1 between the line LN perpendicular to the inclined surface 101A and the center axis AX of the optical fiber as described above, and therefore, is equal to or greater than 46 degrees. Note that the angle θ2 can be adjusted by a change in a box height, for example.

Next, fixing resin such as silicone resin or urethane resin is injected into the box in an uncured state, and as illustrated in FIG. 10B, the multiple optical fibers 101 fixed by the fixing resin 102X in a cured state are taken out of the box 200. Next, as indicated by a chain double-dashed line of FIG. 10C, the fixing resin 102X and the multiple optical fibers 101 are cut parallel to a bottom surface of the fixing resin 102X from a predetermined height position of the fixing resin 102X. Thereafter, a cut surface is polished, and accordingly, the light introduction device 100 as illustrated in FIG. 9 is obtained.

### <Sterilization Method by Sterilization System>

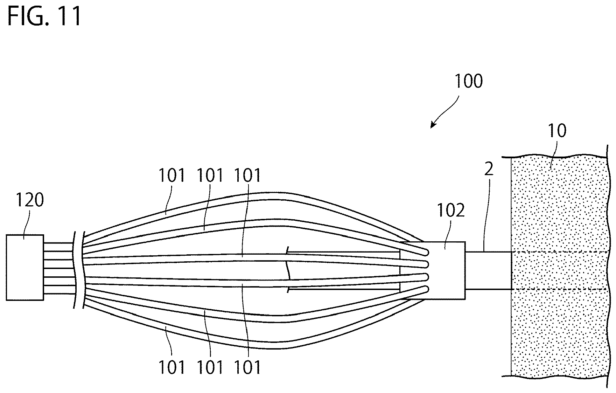

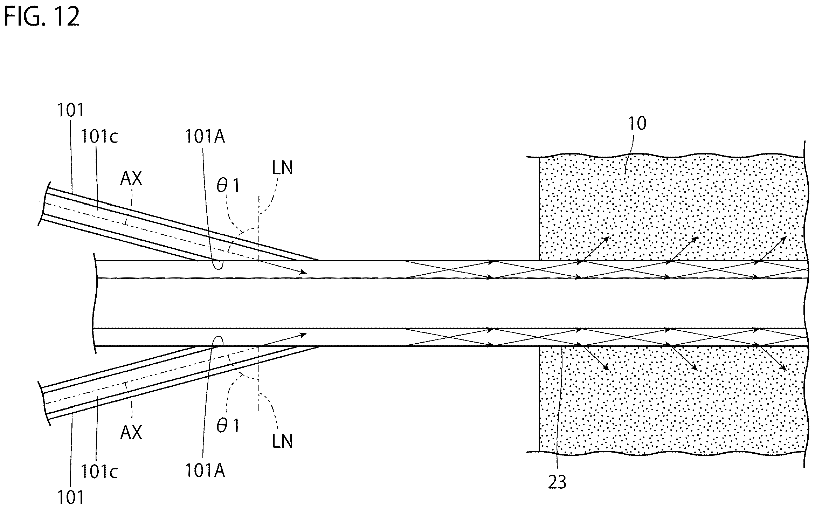

Next, a sterilization method by the sterilization system of the present embodiment will be described with reference to FIGS. 11 and 12. FIG. 11 is a view of the sterilization system attached to the light introduction device 100. FIG. 12 is a sectional view of a light propagation state in the sterilization system of the present embodiment. Note that in FIG. 12, hatching of the sections of the catheter 2 and the optical fibers 101 is omitted for the sake of easy understanding of the light propagation state.

As illustrated in FIG. 11, in the sterilization system 1 of the present embodiment, the fixing member 102 is wound around the outer surface of the catheter 2, and in this manner, the light introduction device 100 is attached to the catheter 2. The fixing member 102 has the flexibility as described above, and therefore, even in a state in which the catheter 2 is placed in a human body, the light introduction device 100 can be attached onto the outer surface of the catheter 2. Note that the fixing member 102 wound around the outer surface of the catheter 2 may be fixed with a predetermined fixing tool. Note that in the present embodiment, the light introduction device has the light source unit 120 configured to introduce the ultraviolet light to the cores of the optical fibers 101, and the light source unit 120 is connected to each optical fiber 101.

In a state in which the light introduction device 100 is provided at a section of the catheter 2 arranged outside the human body as described above, each optical fiber 101 is arranged closer to the catheter 2 toward an opening end of the catheter 2 on a human body inner side. That is, in a case where the opening end of the catheter 2 on the human body inner side is one opening end, each optical fiber 101 is arranged closer to the catheter 2 as a medical conduit toward one opening end side.

As illustrated in FIG. 12, in a state in which the light introduction device 100 is attached, the inclined surface 101A of each of the multiple optical fibers 101 positioned on the substantially same plane as that of the arrangement surface 102A of the fixing member 102 contacts the outer surface of the catheter 2. Thus, in the present embodiment, the outer surface of the catheter 2 as the medical conduit and the inclined surfaces 101A of the optical fibers 101 face each other in parallel. Moreover, the direction of arrangement of the inclined surfaces 101A of the multiple optical fibers 101 is substantially coincident with the circumferential direction of the outer surface of the catheter 2.

In this state, the ultraviolet light is introduced into the core 101c of each of the multiple optical fibers 101 fixed to the fixing member 102 from the light source unit 120. The ultraviolet light propagates in the core 101c of each optical fiber 101, and enters the outer surface of the catheter 2 contacting the inclined surface 101A of such an optical fiber 101.

When the entrance angle of light entering the outer surface of the catheter 2 is equal to or greater than 46 degrees, light introduced into a thick portion of the catheter 2 is easily totally reflected on an inner surface of the catheter 2. In the present embodiment, the angle between the line LN perpendicular to the inclined surface 101A of the optical fiber 101 and the center axis AX of the optical fiber is equal to or greater than 46 degrees. Thus, the entrance angle of the ultraviolet light propagating in the core 101c of each optical fiber 101 is equal to or greater than 46 degrees. Thus, large part of the ultraviolet light introduced into the thick portion of the catheter 2 is totally reflected on the inner surface of the catheter 2, and propagates in the thick portion of the catheter 2 toward the opening end 2B on the human body inner side. As described in the first embodiment, the ultraviolet light propagating in the thick portion of the catheter 2 is released from a human tissue arrangement portion 23 of the catheter 2 to a human tissue 10.

As described above, according to the sterilization system of the present embodiment, bacteria interposed between the catheter 2 and the human tissue 10 can be directly irradiated with the ultraviolet light from the inside as in the above-described first embodiment. Thus, as compared to the case of irradiating a human body with ultraviolet light from the outside, bacteria interposed between the human tissue 10 and a conduit portion surrounded by the human tissue 10 can be efficiently sterilized.

Note that the multiple optical fibers 101 of the light introduction device 100 in the present embodiment are fixed to the fixing member 102 having the flexibility, and the inclined surfaces 101A of the optical fibers 101 are arranged at the intervals ITL in the predetermined direction. Thus, the direction of arrangement of the optical fibers 101 becomes coincident with the circumferential direction of the outer surface of the catheter 2 as described above so that the ultraviolet light can be introduced into the thick portion of the catheter 2 from the circumferential direction. Thus, the ultraviolet light can be released toward the human tissue 10 from a circumferential direction of the human tissue arrangement portion 23 of the catheter 2, and as a result, an ultraviolet irradiation area can be more expanded.

Moreover, the multiple optical fibers 101 in the present embodiment are fixed to the fixing member 102 having the flexibility, and the inclined surfaces 101A of the optical fibers 101 are positioned on the same plane as that of the arrangement surface 102A of the fixing member 102. Thus, the fixing member 102 having a predetermined length is wound around the catheter 2, and both end surfaces of the fixing member 102 are bonded to each other. In this manner, the end portions of the optical fibers 101 can substantially closely contact the outer surface of the catheter 2. Thus, as described above, reflection of light emitted from the inclined surfaces 101A on the outer surface of the catheter 2 is substantially eliminated, and therefore, such light can be efficiently introduced into the catheter 2.

Note that at least one of the outer diameter, the fiber number, or the interval ITL of the optical fiber 101 arranged in the predetermined direction is easily differentiated in manufacturing. Thus, even in the case of using the catheters 2 with different outer diameters on site, the detachable light introduction device 100 can be prepared according to use.

### (5) Fifth Embodiment

Next, a fifth embodiment will be described. Note that the same reference numerals are used to represent configurations similar to those described in the first embodiment, and unless otherwise described, overlapping description will be omitted.

### <Configuration of Sterilization System>

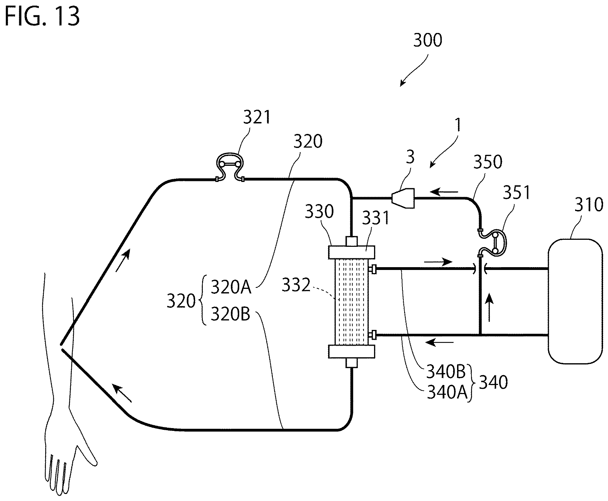

FIG. 13 is a view of an outline configuration of a dialysis device including a sterilization system in the fifth embodiment. That is, the sterilization system 1 of the present embodiment is part of the dialysis device 300. As illustrated in FIG. 13, the dialysis device 300 includes, as main components, a patient monitoring device 310, a blood circuit 320, a dialyzer 330, a dialysate solution flow path 340, and a dialysate solution refilling flow path 350.

The blood circuit 320 is a path allowing passage of blood taken out from a not-shown shunt embedded in a human body, and includes a tube having elasticity. In the present embodiment, the tube forming the blood circuit 320 has a configuration similar to that of the catheter 2 as the medical conduit of the first embodiment. The dialyzer 330 is provided at a middle section of the blood circuit 320. Of the blood circuit 320, an artery-side blood circuit 320A on an inlet port side of the shunt with respect to the dialyzer 330 is provided with a blood pump 321. Note that each of the artery-side blood circuit 320A of the blood circuit 320 and a vein-side blood circuit 320B on an outlet port side of the shunt with respect to the dialyzer 330 may be provided with an air trap chamber configured to remove air from the blood and trap a clot.

The blood pump 321 is a roller pump configured to send, with, e.g., a not-shown rotary roller, the blood to the blood circuit 320 by means of the blood circuit 320, and causes the blood to flow into the blood circuit 320.

The dialyzer 330 is a dialyzing machine configured to remove a waste from the blood, and has a body case 331 with a housing space for housing a dialysate solution and multiple semipermeable membrane tubes 332 arranged in the housing space.

At an input portion of the body case 331, one end of the artery-side blood circuit 320A and one end of each semipermeable membrane tube 332 in the body case 331 are connected to each other. At an output portion of the body case 331, one end of the vein-side blood circuit 320B and the other end of each semipermeable membrane tube 332 in the body case 331 are connected to each other.

The patient monitoring device 310 is coupled to other sections of the body case 331 than the input portion and the output portion through the dialysate solution flow path 340. The dialysate solution flow path 340 is a path allowing passage of the dialysate solution, and includes a tube having elasticity. In the present embodiment, the tube forming the dialysate solution flow path 340 has a configuration similar to that of the catheter 2 as the medical conduit of the first embodiment.

The patient monitoring device 310 is configured to supply and recover the dialysate solution through the dialysate solution flow path 340. Thus, the dialysate solution supplied from the patient monitoring device 310 flows into the housing space of the body case 331 through an upstream-side dialysate solution flow path 340A. On the other hand, the blood of a dialysis patient flows into an inner cavity of each semipermeable membrane tube 332 arranged in the housing space of the body case 331 through the artery-side blood circuit 320A.

Moreover, in the present embodiment, the artery-side blood circuit 320A and the upstream-side dialysate solution flow path 340A are connected to each other through the dialysate solution refilling flow path 350. Specifically, one end of the dialysate solution refilling flow path 350 is connected to a portion of the artery-side blood circuit 320A between the blood pump 321 and the dialyzer 330, and the other end of the dialysate solution refilling flow path 350 is connected to the upstream-side dialysate solution flow path 340A. The dialysate solution refilling flow path 350 is a path allowing passage of the dialysate solution branched from the dialysate solution flow path 340A, and includes a tube having elasticity. In the present embodiment, the tube forming the dialysate solution refilling flow path 350 has a configuration similar to that of the medical conduit of the first embodiment. A dialysate solution refilling pump 351 is provided in the middle of the dialysate solution refilling flow path 350. The dialysate solution refilling pump 351 is a roller pump configured to send, with, e.g., a not-shown rotary roller, the dialysate solution branched from the dialysate solution flow path 340A to the artery-side blood circuit 320A by means of the dialysate solution refilling flow path 350.

The dialysate solution refilling flow path 350 is provided with a light introduction device 3. The light introduction device 3 has a configuration similar to that of the light introduction device 3 in the first embodiment. Thus, as in the light introduction device 3 fixed to the catheter 2 in the first embodiment, the light introduction device 3 of the present embodiment is fixed to the medical conduit forming the dialysate solution refilling flow path 350. Thus, in the present embodiment, the dialysate solution refilling flow path 350 and the light introduction device 3 form the sterilization system 1. Note that in the present embodiment, an emitting-side end portion 50B of a light guide member 50 at the light introduction device 3 faces an opening end of the dialysate solution refilling flow path 350 on an artery-side blood circuit 320A side. Thus, in a case where the opening end of the dialysate solution refilling flow path 350 on the artery-side blood circuit 320A side is one opening end, an opening end of the dialysate solution refilling flow path 350 closer to the dialysate solution flow path 340A is the other opening end, the emitting-side end portion 50B of the light guide member 50 is one end portion, and an entrance-side end portion 50A is the other end portion, one end portion of the light guide member 50 facing one opening end side of the dialysate solution refilling flow path 350 is diagonally inclined toward the dialysate solution refilling flow path 350 as extending toward one opening end side of the dialysate solution refilling flow path 350, and light is entered from the other end side of the light guide member 50 facing the other opening end side of the dialysate solution refilling flow path 350.

### <Sterilization Method by Sterilization System>

Next, a sterilization method by the sterilization system 1 will be described together with operation of the dialysis device 300. In the case of operating the dialysis device 300, the blood pump 321 is operated to send the blood from the above-described shunt to the semipermeable membrane tubes 332 of the dialyzer 330. Moreover, the patient monitoring device 310 supplies the dialysate solution to the housing space of the body case 331 of the dialyzer 330 through the dialysate solution flow path 340A. Thus, in the housing space of the body case 331 of the dialyzer 330, a substance moves between the blood and the dialysate solution through each semipermeable membrane tube 332. Specifically, the waste such as urine toxin in the blood moves into the dialysate solution through the semipermeable membrane tubes 332 according to a concentration gradient. Moreover, components such as red blood cells, white blood cells, and protein in the blood flow in the tubes without penetrating the semipermeable membrane tubes 332. In a case where an electrolyte concentration in the blood is higher than an electrolyte concentration in the dialysate solution, an electrolyte in the blood moves into the dialysate solution through the semipermeable membrane tubes 332 according to a concentration gradient. In a case where the electrolyte concentration in the blood is lower than the electrolyte concentration in the dialysate solution, the electrolyte moves into the blood through the semipermeable membrane tubes 332 according to the concentration gradient. Similarly, in a case where a glucose concentration in the blood is higher than a glucose concentration in the dialysate solution, glucose in the blood moves into the dialysate solution through the semipermeable membrane tubes 332 according to a concentration gradient. In a case where the glucose concentration in the blood is lower than the glucose concentration in the dialysate solution, the glucose moves into the blood through the semipermeable membrane tubes 332 according to the concentration gradient.

The dialysate solution containing the waste moved from the blood is, as waste fluid, recovered from the housing space of the body case 331 by the patient monitoring device 310 through a downstream-side dialysate solution flow path 340B. Moreover, the blood from which the waste has been removed by the dialyzer 330 flows into the blood of the dialysis patient from each semipermeable membrane tube 332 through the vein-side blood circuit 320B.

At this point, in the present embodiment, the dialysate solution refilling pump 351 is operated to send the dialysate solution branched from the dialysate solution flow path 340A to the artery-side blood circuit 320A through the dialysate solution refilling flow path 350. In this manner, the blood sent to the semipermeable membrane tubes 332 of the dialyzer 330 through the shunt is diluted with the dialysate solution.

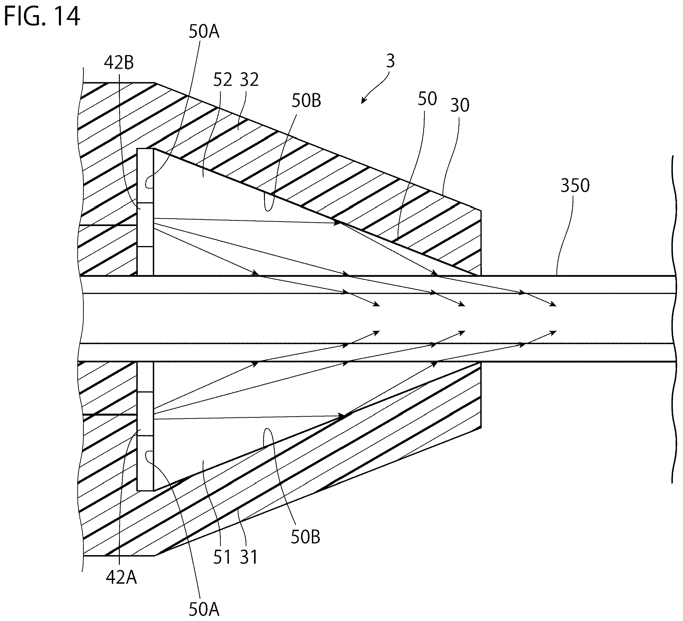

As described above, the dialysate solution refilling flow path 350 is provided with the light introduction device 3. FIG. 14 is a view of an operation state of the light introduction device 3 in the present embodiment as in FIG. 6. As illustrated in FIG. 14, in the light introduction device 3 of the present embodiment, a first LED 42A and a second LED 42B are, as in the light introduction device of the first embodiment, turned on to emit ultraviolet light from the first LED 42A and the second LED 42B. As in the first embodiment, such ultraviolet light enters the light guide member 50 through the entrance-side end portion 50A thereof, and propagates toward the emitting-side end portion 50B of the light guide member 50. Large part of the light propagating to the emitting-side end portion 50B is reflected on the emitting-side end portion 50B, and enters the medical conduit in a state diagonally to a longitudinal direction of the medical conduit forming the dialysate solution refilling flow path 350. The dialysate solution as liquid is introduced into an inner cavity of the medical conduit of the present embodiment. A difference between the refractive index of a thick portion of the medical conduit and the refractive index of the dialysate solution is smaller than a difference between the refractive index of the thick portion of the medical conduit and the refractive index of atmospheric air. Thus, most of the ultraviolet light having entered the medical conduit is released to the dialysate solution as illustrated in FIG. 14. Thus, in a case where bacteria are present in the dialysate solution, these bacteria can be directly irradiated with the ultraviolet light. Note that when the refractive index of the thick portion of the medical conduit is lower than the refractive index of the dialysate solution as the liquid introduced into the inner cavity of the medical conduit, it is preferable because light easily enters the dialysate solution from the medical conduit.

As described above, according to the sterilization system 1 of the present embodiment, the liquid is introduced into the inner cavity of the medical conduit. Such liquid is a substance contacting an inner wall of the medical conduit. Light can propagates in the substance through the medical conduit, and bacteria present in the liquid can be deactivated such that the amount of the bacteria is decreased. Thus, a sterilization effect for the liquid introduced into the human body can be enhanced.

Note that in the present embodiment, the light introduction device 3 is provided at the dialysate solution refilling flow path 350, and the dialysate solution refilling flow path 350 and the light introduction device 3 form the sterilization system 1. However, a location where the light introduction device 3 is provided is not limited to above. For example, the light introduction device 3 may be provided at the dialysate solution flow path 340A, and the dialysate solution flow path 340A and the light introduction device 3 form the sterilization system.

### (6) Sixth Embodiment

Next, a sixth embodiment will be described. Note that the same reference numerals are used to represent configurations similar to those described in the first embodiment, and unless otherwise described, overlapping description will be omitted.

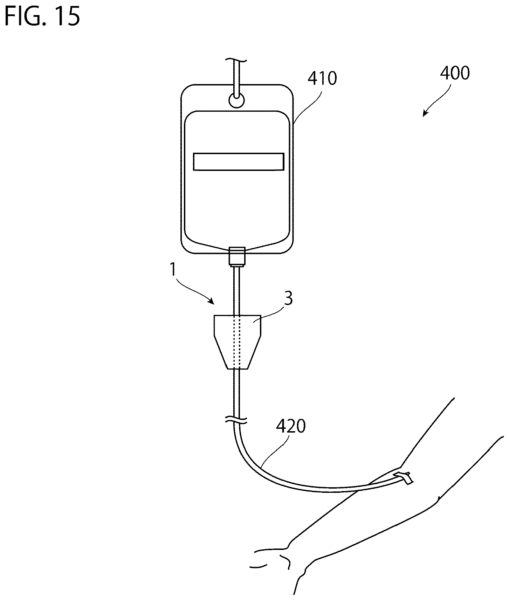

FIG. 15 is a view of an outline configuration of an infusion device including a sterilization system in the sixth embodiment. That is, the sterilization system 1 of the present embodiment is part of an infusion device 400. The infusion device 400 of the present embodiment includes, as main components, an infusion solution bag 410 and an infusion tube 420 as a medical conduit.

The infusion solution bag 410 is filled with a chemical solution. Moreover, one end of the infusion tube 420 is connected to a lower portion of the infusion solution bag 410, and a not-shown infusion needle is provided at the other end of the infusion tube 420. The infusion tube 420 is a path allowing passage of the chemical solution of the infusion solution bag 410, and includes a tube having elasticity. In the present embodiment, the infusion tube 420 has a configuration similar to that of the catheter 2 as the medical conduit of the first embodiment. Thus, the chemical solution in the infusion solution bag 410 is introduced into a human body through the infusion tube. Note that the infusion tube 420 may be provided with an infusion cylinder.

Moreover, in the infusion device 400 of the present embodiment, the infusion tube 420 is provided with a light introduction device 3. The light introduction device 3 has a configuration similar to that of the light introduction device 3 in the first embodiment. Thus, as in the light introduction device 3 fixed to the catheter 2 in the first embodiment, the light introduction device 3 of the present embodiment is fixed to the infusion tube 420. Thus, in the present embodiment, the infusion tube 420 and the light introduction device 3 form the sterilization system 1.

As in the light introduction device of the first embodiment, ultraviolet light is, in the infusion device 400, entered to the infusion tube 420 as the medical conduit from the light introduction device 3 in the middle of infusion. Specifically, as in entrance of the ultraviolet light from the light introduction device 3 to the medical conduit in the fifth embodiment described with reference to FIG. 14, the ultraviolet light is entered from the light introduction device 3 to the infusion tube 420. As in entrance of the ultraviolet light from the medical conduit to the dialysate solution in the fifth embodiment, the ultraviolet light having entered the infusion tube 420 is released from the infusion tube 420 to the chemical solution introduced into an inner cavity of the infusion tube 420. Thus, in a case where bacteria are present in the chemical solution, these bacteria can be directly irradiated with the ultraviolet light. Note that when the refractive index of a thick portion of the infusion tube 420 is lower than the refractive index of the chemical solution as liquid introduced into the inner cavity of the infusion tube 420, it is preferable because light easily enters the chemical solution from the infusion tube 420.

As described above, in the present embodiment, light can propagate, as in the sterilization system 1 of the fifth embodiment, in the chemical solution as a substance contacting an inner wall of the infusion tube 420, and the bacteria present in the chemical solution can be deactivated such that the amount of the bacteria is decreased. Thus, a sterilization effect for the liquid introduced into the human body can be enhanced.

### (7) Variations

The embodiments of the present invention have been described above by way of example, but may be modified as necessary.

For example, in the above-described embodiments, the catheter 2 having the portion where the light transmission for the wavelength band of 270 nm to 340 nm per millimeter in length is equal to or higher than 95% is used as the medical conduit. However, instead of the catheter 2, a catheter having no portion where the light transmission for the wavelength band of 270 nm to 340 nm per millimeter in length is equal to or higher than 95% may be used.

Moreover, in the above-described embodiments, the zone from the opening end 2A of the catheter 2 on the human body outer side to the boundary position between the human tissue arrangement portion 23 and the intracorporeal arrangement portion 21 is applied as the first zone SC1 where the transmission for the ultraviolet light per millimeter in length is equal to or higher than 95%. However, e.g., a first zone may be applied, in which a middle section from the opening end 2A of the catheter 2 on the human body outer side to the human tissue arrangement portion 23 is a start position of the first zone SC1 and the boundary position between the human tissue arrangement portion 23 and the intracorporeal arrangement portion 21 is an end position of the first zone SC1. Alternatively, the end position of the first zone SC1 may be, for example, a middle section of the intracorporeal arrangement portion 21. Alternatively, only the human tissue arrangement portion 23 may be applied as the first zone, for example. As described above, the first zone including the section of the catheter 2 surrounded by the human tissue 10 and the second zone other than the first zone can be applied.

Further, only part of the human tissue arrangement portion 23 may be taken as the first zone. For example, a portion of the human tissue arrangement portion 23 after the external cuff 24A or the internal cuff 24B may be applied as the first zone. Alternatively, the entirety of the catheter 2 may be taken as the first zone, and the second zone may be omitted. In other words, the transmission for the ultraviolet light per millimeter in length may be equal to or higher than 95% at at least part of the section of the catheter 2 surrounded by the human tissue 10.

Note that in a case where the first zone includes at least part of the intracorporeal arrangement portion 21, bacteria in liquid stored in an inner cavity of the intracorporeal arrangement portion 21 can be sterilized such that the amount of the bacteria is decreased.

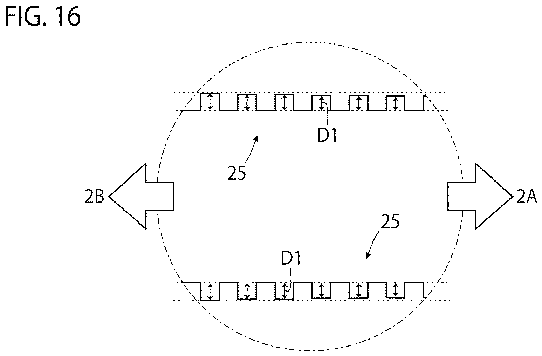

In addition, in the above-described embodiments, the surface of the catheter 2 is not processed, but the following processing may be performed. FIG. 16 is an enlarged view of a portion surrounded by a chain line of FIG. 2, illustrating a state in which the surface of the catheter 2 is processed. For example, as illustrated in FIG. 16, a recessed-raised portion 25 may be provided at the surface of the human tissue arrangement portion 23. Note that the recessed-raised portion 25 may be also provided at the surface of the portion of the human tissue arrangement portion 23 provided with the external cuff 24A and the internal cuff 24B. With this configuration, the amount of light released from the human tissue arrangement portion 23 to the human tissue 10 can be adjusted.

Note that a height difference D1 at the recessed-raised portion 25 is preferably substantially equal to the light source wavelength, or is preferably about ten times as great as the light source wavelength. The height difference D1 may be within a range of 200 nm to 2 µm. Moreover, as illustrated in FIG. 16, in a case where the height difference D1 increases with a distance from the opening end 2A of the catheter 2 on the human body outer side, light can be uniformly released along the longitudinal direction of the catheter 2.

In addition to the height difference D1 or instead of the height difference D1, the density of the recessed-raised portion 25 per unit area may increase with a distance from the opening end 2A of the catheter 2 on the human body outer side. Specifically, in a case where the intensity of light released per square area of 1 cm in length and 1 cm in width is, for example, a predetermined value such as 1 mW, one or both of the height difference and the density increase with a distance from the opening end 2A of the catheter 2 on the human body outer side such that the intensity of light released per square area is the predetermined value even with any distance from the opening end 2A on the human body outer side. Alternatively, one or both of the height difference D1 and the density of the recessed-raised portion 25 at the portion provided with the cuff 24 may be greater than one or both of the height difference D1 and the density of the recessed-raised portion 25 at other portions. With this configuration, the cuff 24 having such tendency that bacteria are easily accumulated at the cuff 24 can be irradiated with the ultraviolet light in a focused manner.

Further, in addition to the recessed-raised portion 25 or instead of the recessed-raised portion 25, multiple reflectors dispersed at the surface of the human tissue arrangement portion 23 may be provided at such a surface. A metal thin film made of aluminum or silver may be utilized as the reflector, and the reflectors may be in a dot pattern. With this configuration, the amount of light released from the human tissue arrangement portion 23 to the human tissue 10 can be adjusted. Note that in the case of providing the reflectors in addition to the recessed-raised portion 25, a predetermined amount of light released from the human tissue arrangement portion 23 to the human tissue 10 can be ensured while the light can more easily propagate to a lumen side of the human body.

In a case where the percentage of a reflection film per unit area of the catheter 2 decreases with a distance from the opening end of the catheter 2 on the human body outer side, these reflectors can ensure the predetermined amount of light released from the human tissue arrangement portion 23 to the human tissue 10 while more easily adjusting light propagation to the lumen side of the human body.

Moreover, in the above-described embodiments, no member configured to trap the ultraviolet light in the catheter 2 is provided, but such a member may be provided. Specifically, one or both of outer and inner surfaces of the extracorporeal arrangement portion 22 are, for example, coated or applied with a member such as a reflection film configured to reflect the ultraviolet light. With this configuration, more ultraviolet light can be guided from the extracorporeal arrangement portion 22 to the human tissue arrangement portion 23.

Further, in the above-described embodiments, no member configured to visually check the ultraviolet light is provided at the catheter 2, but such a member may be provided. Specifically, the outer surface of the extracorporeal arrangement portion 22 is, for example, coated or applied with a light emission member configured to emit visible light by the ultraviolet light. Note that a through-hole penetrating the housing 30 from an outer surface to an inner surface thereof may be provided at the third tube portion PT3 of the housing 30, and the member configured to emit the visible light may be provided in the through-hole.

In addition, in the above-described embodiments, the peritoneal dialysis catheter 2 is applied as the medical conduit. However, other catheters such as a urinary catheter can be applied as the medical conduit. Moreover, a drain may be applied as the medical conduit.

Moreover, in the above-described embodiments, the light introduction device 3 is detachable from the catheter 2. However, the light introduction device 3 may be fixed to the catheter 2. Note that the light introduction device 3 is preferably detachable from the catheter 2, considering that the light introduction device 3 is replaced with the catheter 2 remaining in the human body.

Further, in the above-described embodiments, the housing 30, the light emitter 40, and the light guide member 50 are provided as the components of the light introduction device 3. However, some of the components of the light introduction device 3 may be omitted. For example, the housing 30 may be omitted, and the light guide member 50 attached to the light emitter 40 may be detachable from the catheter 2 in a state in which the end surface of the entrance-side end portion 50A of the light guide member 50 and a light emission surface of the light emitter 40 contact each other. Note that the catheter 2 having the portion where the light transmission for the wavelength band of 270 nm to 340 nm per millimeter in length is equal to or higher than 95% may be the component of the light introduction device 3. Moreover, the third tube portion PT3 of the light introduction device 3 may also serve as the connector 2CN.

In addition, in the above-described embodiments, the light introduction device 3 is provided on the side surface of the end portion of the catheter 2 on the human body outer side, and light is introduced through such a side surface. However, the light introduction device may be, for example, provided at a tip end of the opening end 2A of the catheter 2 on the human body outer side, and light may be introduced into the catheter 2 through the opening end 2A. Alternatively, the light introduction device may be, for example, provided at the middle section of the extracorporeal arrangement portion 22 of the catheter 2, and light may be introduced through a side surface of the middle section. Note that the light introduction device 3 is preferably provided at a spot of the extracorporeal arrangement portion 22 of the catheter 2 apart from an outlet portion of the subcutaneous tunnel by, e.g., 1 cm or longer, considering that the probability of entrance of bacteria through the outlet portion upon, e.g., attachment of the light introduction device 3 to the catheter 2 is reduced.

Moreover, in the above-described embodiments, the ultraviolet light is introduced into the catheter 2 from the light introduction device 3, but visible light may be introduced together with such light. Specifically, in the first embodiment, an LED configured to emit the visible light is provided together with the LEDs 42, and the ultraviolet light and the visible light are emitted from the LED and the LEDs 42 to the entrance-side end portion 50A of the light guide member 50, for example. Moreover, in the second embodiment, the ultraviolet light and the visible light are generated by the light source 144, and these types of light are emitted to the entrance-side end portion 50A of the light guide member 50 through the optical fibers 143A, 143B, for example. Note that other light than the ultraviolet light may be introduced, but the ultraviolet light is preferable considering enhancement of the sterilization ability.

In the above-described fifth and sixth embodiments, the light introduction device 3 of the first embodiment is provided at the medical conduit, but the light introduction device 3 of the second embodiment or the light introduction device 100 of the fourth embodiment may be provided at the medical conduit, for example.

### Reference Signs List

- 1: sterilization system
- 2: catheter
- 3, 100: light introduction device
- 21: intracorporeal arrangement portion
- 22: extracorporeal arrangement portion
- 23: human tissue arrangement portion
- 24: cuff
- 25: recessed-raised portion
- 30: housing
- 40: light emitter
- 50: light guide member
- 101: optical fiber
- 102: fixing member
- 300: dialysis device
- 310: patient monitoring device
- 320: blood circuit
- 330: dialyzer
- 340: dialysate solution flow path
- 350: dialysate solution refilling flow path
- 400: infusion device
- 410: infusion solution bag
- 420: infusion tube

## Claims

1. A light introduction device comprising:
an inner cavity allowing passage of at least one of a substance introduced into a human body or a substance discharged from the human body,
wherein light is introduced from part of an outer surface of a section, which is arranged outside the human body, of a medical conduit having a light-permeable thick portion.

2. The light introduction device according to claim 1, further comprising:
a light guide member surrounding the outer surface of the section of the medical conduit arranged outside the human body,
wherein one end portion of the light guide member facing one opening end side of the medical conduit is diagonally inclined toward the medical conduit as extending toward the one opening end side of the medical conduit, and the light entering from another end side of the light guide member facing an other opening end side of the medical conduit is entered to the medical conduit diagonally to a longitudinal direction of the medical conduit.

3. The light introduction device according to claim 2, wherein
the light guide member and the medical conduit closely contact each other.

4. The light introduction device according to claim 1, further comprising:
an optical fiber allowing entrance of the light from the outer surface of the section of the medical conduit arranged outside the human body,
wherein one end portion of the optical fiber has an inclined surface inclined with respect to a center axis of the optical fiber, and is arranged closer to the medical conduit as extending toward one opening end side of the medical conduit, and
the inclined surface faces an outer surface of the medical conduit.

5. The light introduction device according to claim 4, wherein
an angle between a line perpendicular to the inclined surface and the center axis of the optical fiber is equal to or greater than 46 degrees, and
the outer surface of the medical conduit and the inclined surface are parallel to each other.

6. The light introduction device according to claim 4 or 5, further comprising:
a fixing member configured to fix multiple optical fibers,
wherein the fixing member has an arrangement surface arranged on the outer surface of the medical conduit, and covers one end portion of each of the multiple optical fibers such that an inclined surface of each of the multiple optical fibers is exposed from an arrangement surface side.

7. The light introduction device according to claim 6, wherein
the fixing member has flexibility, and
the inclined surfaces of the multiple optical fibers are arranged at intervals in one direction.

8. The light introduction device according to any one of claims 4 to 7, wherein
the inclined surface of each optical fiber closely contacts the outer surface of the medical conduit.

9. The light introduction device according to any one of claims 1 to 8, wherein the light has a peak wavelength in a wavelength band of 270 nm to 340 nm.

10. A sterilization system comprising:
a medical conduit including an inner cavity allowing passage of at least one of a substance introduced into a human body or a substance discharged from the human body and including a light-permeable thick portion; and
the light introduction device according to any one of claims 1 to 9.

11. The sterilization system according to claim 10, wherein
liquid is introduced into the inner cavity of the medical conduit.

12. The sterilization system according to claim 10 or 11, wherein
the medical conduit is arranged from an outside to an inside of the human body.

13. The sterilization system according to claim 12, wherein
at least part of a section of the medical conduit surrounded by a human tissue is configured such that a light transmission for a wavelength band of 270 nm to 340 nm per millimeter in length is equal to or greater than 95%.

14. The sterilization system according to claim 13, wherein
the medical conduit includes a first zone including the section surrounded by the human tissue, and a second zone on an opposite side of the first zone from the outside of the human body,
the first zone is configured such that the light transmission for the wavelength band of 270 nm to 340 nm per millimeter in length is equal to or greater than 95%, and
the second zone is configured such that the light transmission for the wavelength band of 270 nm to 340 nm per millimeter in length is less than 95%.

15. The sterilization system according to claim 14, wherein
the first zone includes at least part of the section arranged outside the human body.

16. The sterilization system according to claim 12, wherein
a recessed-raised portion is provided at part of a surface of a section of the medical conduit surrounded by a human tissue.

17. The sterilization system according to claim 16, wherein
a height difference at the recessed-raised portion increases with a distance from an opening end of the medical conduit on the outside of the human body.

18. The sterilization system according to claim 12, wherein
at part of a surface of a section of the medical conduit surrounded by a human tissue, multiple reflectors dispersed on the surface are provided.

19. The sterilization system according to claim 18, wherein
a percentage of the reflectors per unit area of the medical conduit decreases with a distance from an opening end of the medical conduit on the outside of the human body.
